# EUROPEAN PATENT APPLICATION

(11) **EP 4 293 108 A1**
(43) Date of publication of application: **20.12.2023**
(21) Application number: 22766934.8
(22) Date of filing: 02.03.2022
(51) Int. Cl.: C12N 5/071, C12N 15/09, C12Q 1/02, C12Q 1/6813, C12Q 1/6851, C12Q 1/686, C12Q 1/6876

(54) **METHOD FOR DETECTING OR QUANTIFYING PHOTOAGED CELLS, APPLICATION OF SAME, AND METHOD FOR PREPARING PHOTOAGED CELLS**

(30) Priority: 09.03.2021 JP 2021037357
(71) Applicant: Ushio Denki Kabushiki Kaisha, Tokyo 100-8150 (JP)
(72) Inventor: SHIMADA,Shun, Tokyo 100-8150 (JP)
(74) Representative: Maiwald GmbH
(86) International application number: PCT/JP2022/008772
(87) International publication number: WO 2022/190975

(57) **Abstract**

Provided is a method capable of efficiently preparing a photoaged cell. Also provided is a novel cell marker that can be used for detection, quantification, or sorting out of a photoaged cell. The present disclosure includes a method for preparing a photoaged cell, including the step of: irradiating a cell with light having an emission peak within a wavelength range of 300 to 315 nm, and a method for detecting or quantifying a photoaged cell, including the step of: detecting or quantifying expression of at least one gene selected from the group consisting of GPR17, CD34, GABRR1, OR2AG2, CMKLR1, CDH19, CD93, AVPR2, CCR7, and OXGR1 in a cell.

## Description

### TECHNICAL FIELD

The present invention relates to a method for detecting or quantifying a photoaged cell, a method for sorting out a photoaged cell, which is an application of the method, a method for screening for a substance that suppresses proliferation or development of a photoaged cell or a substance that reduces a photoaged cell, a kit for detecting or quantifying a photoaged cell or sorting out a photoaged cell, and a method for preparing a photoaged cell.

### BACKGROUND ART

When cells are aged, senescence-associated secretory phenotype (SASP) factors such as inflammatory cytokines, chemokines, extracellular matrix-degrading enzymes, and platelet-derived growth factor (PDGF) are released, and surrounding cells are further adversely affected, so that aging proceeds. Development of pharmaceuticals for preventing or improving such a state of cellular aging has been an issue

Cellular aging is roughly classified into two types: photoaging caused by ultraviolet irradiation and natural aging caused by increasing age or other factors.

It is said that 80% of the aging of skin cells is due to photoaging. Photoaging of the skin frequently occurs in a part exposed to sunlight such as the face, neck, and backs of the hand, and causes spots, wrinkles (particularly deep wrinkles), sagging, thickening of the skin, yellow or brown coloration of the skin and the like. On the other hand, natural aging of the skin proceeds regardless of sunlight irradiation. In natural aging, spots are hardly formed, and fine and shallow wrinkles are formed. Also, in natural aging, the skin tends to shrink, and the skin is not colored.

As described above, photoaging frequently occurs in the skin related to the development, and impairs the aesthetic appearance. Therefore, development of external compositions such as cosmetics and quasi-drugs for preventing or improving the state of photoaging have been actively pursued.

To screen for an active ingredient that prevents or improves the state of photoaging in vitro, photoaged cells need to be prepared first. As a method for preparing a photoaged skin tissue, Patent Documents 1 and 2 disclose methods of photoaging human skin that has been transplanted into a mouse or mouse skin by irradiating them with UVB or UVA. Patent Document 2 discloses a method including: contacting mouse fibroblast cultures with a test compound; exposing the fibroblast cultures to UVB, UVA or sunlight-like light; measuring the human elastin promoter activity in the resulting mouse fibroblast cultures, and identifying a compound that reducing the measured human elastin promoter activity compared to control mouse fibroblast cultures as a compound that reduces photo-damage of the skin.

As a method for aging cultured cells, various methods have been reported, such as natural aging by culturing for 40 days or more, aging by DNA damage caused by irradiation with γ rays or the like, UV irradiation, and by exposure to hydrogen peroxide (H₂O₂) or cigarette smoke.

### PRIOR ART DOCUMENT

### PATENT DOCUMENTS

Patent Document 1: JP-A-2007-167060
Patent Document 2: JP-T-2002-542764

### SUMMARY OF THE INVENTION

### PROBLEMS TO BE SOLVED BY THE INVENTION

However, the cells that have undergone treatments other than light irradiation are likely to differ from actual photoaged cells. In addition, if ultraviolet irradiation is used to produce photo-aged cells, a certain number of cells will die if the irradiation dose is high, resulting in a low yield, and if the dose is low, the production process will take time or photo-aging will be insufficient, resulting in a low production efficiency.

Further, screening for substances that prevent or improve photoaging requires sorting out of photoaged cells from normal cells in order to prepare photoaged cells of high purity or to identify the effectiveness. As a means capable of discriminating aged cells, a means of detecting senescence markers such as senescence-associated β-galactosidase (SA-β-Gal) and cyclin dependent kinase inhibitor (p16) is known. However, these markers are all intracellular markers, which makes sorting of living cells not easy. Further, no photoaged cell-specific markers have been known so far.

Therefore, a first object of the present disclosure is to provide a method capable of efficiently preparing a photoaged cell. A second object of the present disclosure is to provide a novel cell marker that can be used for detection, quantification, or sorting out of a photoaged cell.

### MEANS FOR SOLVING THE PROBLEMS

One embodiment of the present disclosure includes a method for preparing a photoaged cell, the method including:
irradiating a cell with light having an emission peak within a wavelength range of 300 to 315 nm.

The preparation method can further include the step of detecting or quantifying expression of at least one gene selected from the group consisting of GPR17, CD34, GABRR1, OR2AG2, CMKLR1, CDH19, CD93, AVPR2, CCR7, and OXGR1 in the cell that has been irradiated with light.

The preparation method can further include the step of sorting out a photoaged cell.

One embodiment of the present disclosure includes a method for detecting or quantifying a photoaged cell, the method including:
detecting or quantifying expression of at least one gene selected from the group consisting of GPR17, CD34, GABRR1, OR2AG2, CMKLR1, CDH19, CD93, AVPR2, CCR7, and OXGR1 in a cell.

One embodiment of the present disclosure includes a method for sorting out a photoaged cell, the method including:
sorting out a cell positive for expression of at least one gene selected from the group consisting of GPR17, CD34, GABRR1, OR2AG2, CMKLR1, CDH19, CD93, AVPR2, CCR7, and OXGR1.

One embodiment of the present disclosure includes a method for screening a substance that suppresses proliferation or development of a photoaged cell or a substance that reduces photoaged cells the method including:
bringing a cell into contact with at least one candidate substance;
detecting or quantifying expression of at least one gene selected from the group consisting of GPR17, CD34, GABRR1, OR2AG2, CMKLR1, CDH19, CD93, AVPR2, CCR7, and OXGR1 in the cell that has been brought into contact with the at least one candidate substance; and
selecting a substance that suppresses proliferation or development of a photoaged cell or a substance that reduces a photoaged cell from the at least one candidate substance according to information obtained by the detection or quantification of expression of the at least one gene.

In the detection or quantification method, the sorting out method, and the screening method, in one embodiment, the detection or quantification of the at least one gene is detection or quantification of proteins encoded by the at least one gene.

Because these proteins are presented on the cell surface, a photoaged cell can be sorted out in a living state by detecting or quantifying the proteins on the cell surface and the sorted-out cell can be used for screening, which is preferable.

One embodiment of the present disclosure includes a kit for detecting, quantifying, or sorting out a photoaged cell, including at least one selected from the group consisting of (a) to (c) below:
(a) a primer set capable of amplifying cDNA of at least one gene selected from the group consisting of GPR17, CD34, GABRR1, OR2AG2, CMKLR1, CDH19, CD93, AVPR2, CCR7, and OXGR1;
(b) a probe that specifically hybridizes with mRNA or cDNA of at least one gene selected from the group consisting of GPR17, CD34, GABRR1, OR2AG2, CMKLR1, CDH19, CD93, AVPR2, CCR7, and OXGR1; and
(c) a substance capable of specifically binding to a protein encoded by at least one gene selected from the group consisting of GPR17, CD34, GABRR1, OR2AG2, CMKLR1, CDH19, CD93, AVPR2, CCR7, and OXGR1.

The substance capable of specifically binding to a protein is preferably an antibody or an antibody fragment.

### EFFECT OF THE INVENTION

According to the method for preparing a photoaged cell of the present disclosure, a photoaged cell can be efficiently prepared.

According to the detection or quantification method and the kit of the present disclosure, a photoaged cell can be easily detected or quantified. And then, the photoaged cell can be easily sorted out by applying the detection or quantification method to the sorting out method. Further, the screening method of the present disclosure, to which the detection or quantification method is applied, can efficiently select a substance that suppresses proliferation or development of a photoaged cell or a substance that reduces a photoaged cell.

### BRIEF DESCRIPTION OF THE DRAWINGS

Fig. 1 is a schematic diagram of the method for preparing a photoaged cell used in each Test Example.
Fig. 2 is microscopic images of normal human epidermal keratinocytes (NHEK) that have been irradiated with ultraviolet rays at various irradiation doses obtained in Test Example 1.
Fig. 3 is a graph comparing mRNA amounts of various genes of NHEK subjected to each treatment of no ultraviolet irradiation treatment (UN), 80 mJ/cm² ultraviolet irradiation (U80), and 50-day subculture treatment (P50) obtained by RNA-Seq analysis of Test Example 2. The vertical axis represents a normalized read count.
Fig. 4 is Hoechst stained images (Hoechst), anti-GPR17 antibody stained images (anti-GPR17), and superimposed images thereof (Merge) of NHEK (UV (-)) without ultraviolet irradiation treatment or NHEK subjected to ultraviolet irradiation treatment at 80 mJ/cm² (UV (+) -1 and UV (+) -2, which were both subjected to the same treatment) in Test Example 3.
Fig. 5 is microscopic images of normal human dermal fibroblasts (NHDF) treated with ultraviolet irradiation at various irradiation doses in Test Example 4.

### MODE FOR CARRYING OUT THE INVENTION

In the present specification, "photoaged cell" generally refers to a living cell that has become aged by ultraviolet irradiation, and has not become cancerous. The photoaged cell is not particularly limited, but has, for example, at least one of the characteristics (1) to (3) below, and preferably has all characteristics of (1) to (3) below:
(1) being β-galactosidase (SA-β-Gal) or cyclin dependent kinase inhibitor (p16) positive;
(2) arrest or significant reduction of cell division is observed; and
(3) cell hypertrophy.

In the present specification, examples of "expression of gene" include a presence of mRNA which is a transcription product of the gene and a presence of a protein that is a translation product of the gene.

In the present specification, "detection" refers to qualitative discrimination of the presence or absence of a substance.

### [Method for preparing photoaged cell]

One embodiment of the present disclosure includes a method of preparing a photoaged cell, the method including: irradiating a cell with light having an emission peak within a wavelength range of 300 to 315 nm.

### (Cell)

The organism from which the cell is derived and the type of the cell are not particularly limited as long as the cell is a living cell, but a cell that has not become cancerous is preferable.

Examples of the organism from which the cell is derived include vertebrates (mammals, reptiles, birds, amphibians, and fish) and invertebrates. Among them, the organism from which the cell is derived is preferably mammals (primates such as, for example, a human, a chimpanzee, a rhesus monkey, and a green monkey; rodents such as a mouse, a rat, and a guinea pig; a horse; a bovine; a sheep; a goat; a canine; a feline and the like), and more preferably a human.

Examples of the type of the cell include a cell of the skin. Specific examples of the cell of the skin include epidermal cells such as a keratinocyte, a pigmented cell (melanocyte), a Langerhans cell, and a Merkel cell; dermal cells such as a fibroblast; adipose tissue cells such as an adipocyte; and hair follicle cells such as a hair papilla cell, a hair matrix cell, an inner root sheath cell, and an outer root sheath cell. Among them, as the type of the cell, epidermal cells or dermal cells are preferable, and a keratinocyte or a fibroblast is more preferable.

Examples of the form of the cell to be irradiated with light include cultured cells such as a primary cultured cell and a cell line; cells in a living body (in an organ or a tissue of a living body); and cells in an organ or a tissue separated from a living body, and among them, cultured cells are preferable.

The cell to be irradiated with light can be, for example, a cell that have undergone induced differentiation from artificial or biogenic stem cells such as an induced pluripotent (iPS) cell, an ES cell, a mesenchymal stem cell, a hematopoietic stem cell, and a neural stem cell; genetic modification; or genome editing.

### (Light irradiation step)

Irradiation with light is preferably performed on cultured cells that have not reached confluence such as sub-confluent cultured cells.

The cells are preferably immersed in a liquid to prevent drying or the like at the time of light irradiation. The liquid is not particularly limited as long as the liquid is not cytotoxic and does not cause cell rupture. Examples of such a liquid include phosphate buffered saline (PBS); Good buffer (HEPES and the like); and one or more components selected from buffer components such as equilibrium salts of Hanks, Ringer, Earle, Gey, Puck, Eagle, Rinaldini, or Tyrode. The liquid preferably does not contain a component that absorbs ultraviolet rays having a wavelength of 300 nm to 315 nm (for example, proteins, nucleic acids, dyes such as phenol red).

The temperature for the cells at the time of light irradiation is, for example, 20 to 42°C, 25 to 40°C, or 30 to 37°C. When the irradiation time is short, the irradiation can be performed in the atmosphere, but a chamber (for example, a CO₂ incubator) in which the CO₂ concentration and the humidity can be appropriately adjusted can be used according to the irradiation time, the irradiation conditions and the like.

The light has an emission peak in a wavelength range of 300 to 315 nm, preferably in a wavelength range of 300 to 310 nm. Such a wavelength component can promote photoaging of cells with reduced cytotoxicity. In a specific embodiment, the wavelength of the emission peak is 308 nm.

The light source of the light having the emission peak include, for example, an excimer discharge lamp (such as XeCl excimer discharge lamp), a fluorescent lamp (such as one in which a gadolinium-activated phosphor material is used), and an LED (for example, an LED element having an emission peak of 312 nm or more or 313 nm or more and 315 nm or less, and a full width at half maximum of 20 nm or less and the like, described in WO 2018/142630). Among them, an excimer discharge lamp or an LED can be suitably used as a light source from the advantages that the risk of heating of cells associated with light irradiation is small and the spectrum is stabilized immediately after lighting.

For example, an optical filter can be applied to the light source. Examples of such an optical filter include one made of borosilicate glass whose main component is silicon oxide, boric acid, and sodium oxide, and the thickness thereof is, for example, 1 to 3 mm. For example, anhydrous, oxygen-free aluminum fluoride glass can also be used. Specifically, the anhydrous, oxygen-free aluminum fluoride glass has a composition in which the contents of BaF₂, CaF₂, and AlF₃ are in the range of 14.00 to 24.00 mol%, in the range of 28.25 to 38.25 mol%, and in the range of 37.25 to 47.25 mol%, respectively, one selected from YF₃, SrF₂, and LaF₃ is contained, when YF₃ is contained, the content thereof is 2.5 to 20 mol%, when SrF₂ is contained, the content thereof is 2.5 to 7.5 mol%, when LaF₃ is contained, the content thereof is 2.5 to 7.5 mol%, and Ce is contained, and no water or oxygen is contained. The content of Ce is preferably 1 to 10 at.%.

The irradiation dose of the light to the cells can be appropriately changed according to the organism from which the cells are derived and the type of the cells, the desired degree of photoaging and the other factors, but from the viewpoint of enhancing the degree of photoaging, the irradiation dose is, for example, 20 mJ/cm² or more, more preferably 40 mJ/cm² or more, still more preferably 60 mJ/cm² or more, and still more preferably 80 mJ/cm² or more.

The irradiation dose of the light to cells is, for example, 200 mJ/cm² or less, preferably 180 mJ/cm² or less, more preferably 150 mJ/cm² or less, and still more preferably 120 mJ/cm² or less or 100 mJ/cm² or less from the viewpoint of reducing cytotoxicity.

In the present specification, the "irradiation dose of light is an irradiation dose calculated from the distance from a light source to cells and the irradiation energy of the light source (theoretical irradiation dose in a case where air exists from the light source to the cells), or an actual measured irradiation dose determined from the irradiation energy of the light detected by placing a detector at the location of the cells.

The irradiation time of the light to the cells can be appropriately adjusted according to the irradiation dose of the light source, the irradiation intensity, the organism from which the cells are derived and the type of the cells, the degree of photoaging of cells to be prepared, the distance between the light source and cells and the other factors, and can be, for example, 0.01 to 60 seconds, 0.1 to 30 seconds, 0.5 to 20 seconds, 1 to 10 seconds, or 2 to 6 seconds.

### (Step of culturing cell)

The present preparation method can further include a step of culturing the cells to recover the cells from injury after the irradiation with the light. Examples of the medium used for the culture can include MEM, α-MEM, DMEM, IMEM, RPMI-1640, Ham's F-12, and dedicated media for various cells. The medium can further contain one or more components selected from the followings: serums such as fetal bovine serum (FBS), horse serum, and human serum; additives such as sodium bicarbonate, sodium pyruvate, L-glutamine, and L-alanyl-L-glutamine; PBS; Good buffer (HEPES and the like); and buffer components such as equilibrium salts including Hanks, Ringer, Earle, Gey, Puck, Eagle, Rinaldini, or Tyrode.

The culture time can be, for example, 1 hour or more, 3 hours or more, 6 hours or more, 12 hours or more, 1 day or more, 2 days or more, or 3 days or more from the viewpoint of promoting cell recovery, and can be 14 days or less, 10 days or less, 7 days or less, or 5 days or less.

### (Other steps)

The present preparation method can further include a separation step for separating individual cells. The separation of the cells includes, for example, physical treatment such as detachment and agitation; treatment with salts such as sodium chloride; treatment with surfactants such as SDS and Triton-X 100; and treatment with enzymes such as dispase and a protease.

The present preparation method can further include a step of sorting out a cell in which photoaging has been induced through light irradiation. In one embodiment, the sorting out step includes, for example, the step described in the method for sorting out described below.

### [Method for detecting or quantifying photoaged cell]

One embodiment of the present disclosure includes a method for detecting or quantifying a photoaged cell, the method including: detecting or quantifying expression of at least one gene selected from the group consisting of GPR17, CD34, GABRR1, OR2AG2, CMKLR1, CDH19, CD93, AVPR2, CCR7, and OXGR1 in a cell.

The cell includes, for example, the cells described in [Method for manufacturing photoaged cell] above.

GPR17 (G protein-coupled receptor 17), which belongs to the G-protein-coupled receptor (GPCR) superfamily, is a gene encoding a cysteinyl leukotriene receptor. For example, the Gene ID of NCBI (URL: https://www.ncbi.nlm.nih.gov/) of human GPR17 is 2840.

CD34 is a gene encoding a transmembrane phosphoglycoprotein. For example, the NCBI Gene ID of human CD34 is 947.

GABRR1 (gamma-aminobutyric acid type A receptor subunit rho1) is a gene encoding a γ-aminobutyric acid (GABA) receptor, which is a ligand-dependent chloride channel. For example, the NCBI Gene ID of human CD34 is 2569.

OR2AG2 (olfactory receptor family 2 subfamily AG member 2), which belongs to the GPCR superfamily, is a gene encoding an olfactory receptor. For example, the NCBI Gene ID of human OR2AG2 is 338755.

CMKLR1 (chemerin chemokine-like receptor 1) is a gene encoding a GPCR whose ligand is a chemotactic adipokine chemerin. For example, the NCBI Gene ID of human CMKLR1 is 1240.

CDH19 (cadherin 19) is a gene encoding cadherin 19, which is one of cadherins. For example, the NCBI Gene ID of human CDH19 is 28513.

CD93 is a gene encoding a C-type lectin transmembrane receptor. For example, the NCBI Gene ID of human CD93 is 22918.

AVPR2 (arginine vasopressin receptor 2), which belongs to the GPCR superfamily, is a gene encoding vasopressin receptor type 2. For example, the NCBI Gene ID of human AVPR2 is 554.

CCR7 (C-C motif chemokine receptor 7) is a gene encoding a GPCR whose ligands are the chemokines CCL19 and CCL21. For example, the NCBI Gene ID of human CCR7 is 1236.

OXGR1 (oxoglutarate receptor 1) is a gene encoding a GPCR belonging to the oxoglutarate receptor family within the GPCR superfamily. For example, the NCBI Gene ID of human OXGR1 is 27199.

The above 10 genes for non-human cells correspond to orthologs of these human genes, and can be obtained, for example, by searching with NCBI Gene by entering the names of the above 10 genes in a query.

The detection or quantification of the expression of the genes is performed, for example, by detecting or quantifying an mRNA that is a transcription product of the genes, or by detecting or quantifying a protein that is a translation product of the genes. The mRNA and protein can include one or more splice variants or processing products.

For detection or quantification of the mRNA, the mRNA is extracted from the cells, and in some cases reverse-transcribed to cDNA and is subjected to amplification reaction by PCR method, and then, for example, the following means can be used.
(a) the mRNA or cDNA of the genes or the amplified product thereof is detected or quantified by a probe that specifically hybridizes to the mRNA or cDNA or the amplified product. Specific examples thereof include Northern blotting and array analysis such as DNA chip analysis.
(b) cDNA is optionally amplified and then subjected to RNA-Seq analysis.
(c) Quantitative PCR (qPCR) is performed using the cDNAs as a template.

For detection or quantification of a protein that is a translation product of the genes, the following means can be used, for example.
(A) By binding a substance that specifically binds to the protein presented on a cell (e.g. an antibody or an antibody fragment, or an aptamer whose antigen is the protein), and the cell is stained directly or with a secondary antibody or the like to detect or quantify the protein.
   As the proteins encoded by the 10 genes are exposed on the cell surface, the means can be applied to living cells. Such means specifically include, for example, detection or quantification by fluorescence in situ hybridization (FISH), flow cytometry or the like.
(B) The protein is extracted from the cell, and the protein is detected or quantified by ELISA method, dot blot method, Western blot method or the like.

For example, one of the present embodiments includes detecting or quantifying the expression of GPR17 gene.

For example, GPR17 has two isoforms, a Long form and a Short form, which differ in the length of N-terminal portions due to a difference in splicing (367 and 339 amino acid residues in humans, respectively; see, for example, American Journal of Physiology, 2009, Vol. 297, Issue 4, C1029-C1040). For detection or quantification of the expression of the gene, the mRNA or the protein of any one or both of short form and long form can be used as a target for detection or quantification. In one embodiment, the detection or quantification of the expression of GPR17 gene is performed by detecting or quantifying mRNA or the protein of Long form of GPR17 gene.

Among the information obtained by the present method, the quantitative information includes, for example, the expression level itself of the genes per a cell population with a specific number of cells, the number of photoaged cells obtained by conversion from the expression level, the number of cells in which the expression of the genes is detected, and the number of cells in which the expression level is not less than a specific value.

### [Method for sorting out photoaged cell]

One embodiment of the present disclosure includes a method for sorting out a photoaged cell, the method including: sorting out a cell positive for expression of at least one gene selected from the group consisting of GPR17, CD34, GABRR1, OR2AG2, CMKLR1, CDH19, CD93, AVPR2, CCR7, and OXGR1.

The cell includes the cell described in [Method for manufacturing photoaged cell] above.

In the present specification, a cell positive for expression of at least one gene refers to a cell in which the expression of the at least one gene is detected, or a cell in which the expression level of not less than a specific threshold value is observed.

In addition, in the present specification, a cell negative for expression of at least one gene refers to a cell in which the expression of the at least one gene is not detected, or a cell in which the expression level is not more than a specific threshold value.

The threshold value is set as appropriate according to the organism from which the cells are derived and the type of the cells, the means for detecting or quantifying the gene expression used and the other factors. For example, the threshold value can be set so that the cells that have undergone photoaging are determined to be positive and the cells that have not undergone photoaging are determined to be negative after preparing the cells that have undergone photoaging and the cells that have not undergone photoaging in advance.

The means for detecting or quantifying the expression of at least one gene include the means described in the section of [Method for detecting or quantifying a photoaged cell] above. Among them, it is particularly preferred to use the means of the above (A), in which a substance that specifically binds to the protein, which is presented on a living cell and encoded by the gene above, is bound to the protein, and the cell is stained in a living state directly or with a secondary antibody or the like to detect or quantify the protein. According to the means, cells can be sorted out in a living state.

In one embodiment, sorting out of cells is performed by flow cytometry. In flow cytometry, for example, the expression of the gene is detected or quantified using an antibody or antibody fragment specific to the protein encoded by the gene.

The present sorting method can be used, for example, without particular limitation, (1) to increase the proportion of photoaged cells to the total number of cells in the preparation of a photoaged cell, or (2) to count cells positive or negative for the expression of the at least one gene above.

[Method for screening for substance that suppresses proliferation or development of photoaged cell or substance that reduces photoaged cell]

One embodiment of the present disclosure includes a method for screening a substance that suppresses proliferation or development of photoaged cells or a substance that reduces photoaged cells, the method including:
bringing a cell into contact with at least one candidate substance;
detecting or quantifying expression of at least one gene selected from the group consisting of GPR17, CD34, GABRR1, OR2AG2, CMKLR1, CDH19, CD93, AVPR2, CCR7, and OXGR1 in the cell that has been brought into contact with the at least one candidate substance;
detecting or quantifying a photoaged cell; and
selecting a substance that suppresses proliferation or development of photoaged cells or a substance that reduces a photoaged cell from the at least one candidate substance according to information obtained by the detection or quantification of expression of the at least one gene.

The suppression of the proliferation of a photoaged cell or the reduction of a photoaged cell can be achieved by, for example, the candidate substance damaging a photoaged cell or improving the aging state of a photoaged cell, but the mechanism of action of the candidate substance is not particularly limited to these.

The reduction of the development of a photoaged cell includes making that a cell less susceptible to photoaging by a prior contact with the candidate substance. Making a cell less susceptible to photoaging is synonymous with, for example, preventing or halting of photoaging of a cell.

### (Step of bringing cell into contact with candidate substance)

The means for bringing a cell into contact with at least one candidate substance include, for example, adding the at least one candidate substance to a medium, and administering the at least one candidate substance to a living body (for example, excluding humans), an organ, or a tissue containing a cell or making a living body (for example, excluding humans), an organ, or a tissue containing a cell ingest the at least one candidate substance.

The candidate substance can be a single substance or a combination of two or more different substances. The candidate substance is, for example, a substance that is a candidate for active ingredients in pharmaceuticals, quasi-drugs, cosmetics, or functional foods.

The cell includes, for example, those described in the section of [Method for detecting or quantifying photoaged cell].

In one embodiment, the cell is a photoaged cell. Such an embodiment is suitable for selecting a substance that suppresses the proliferation of a photoaged cell or reduces a photoaged cell.

In another embodiment, the cell is a cell that has not undergone photoaging. Such an embodiment is suitable for selecting a substance that reduces the development of a photoaged cell (prevents photoaging).

After the cell is brought into contact with the at least one candidate substance, the present embodiment can further include a step of culturing and a step of promoting photoaging of the cell depending on the purpose of the selection, cytotoxicity of the substance and the like. The step of promoting photoaging of the cell include the method described in [Method for preparing photoaged cell] above.

One embodiment further includes a step of promoting photoaging of a cell following contacting the cell, which has not undergone photoaging, with the at least one candidate substance. Such an embodiment is suitable for selecting a substance that reduces the development of a photoaged cell (prevents photoaging).

### (Step of detecting or quantifying photoaged cell)

Specific embodiments of the method for detecting or quantifying a photoaged cell include those described in [Method for detecting or quantifying photoaged cell] above.

### (Step of selecting substance that suppresses proliferation or development of photoaged cell or substance that reduces photoaged cell)

In this step, a substance that suppresses proliferation or development of a photoaged cell or a substance that reduces a photoaged cell is selected from at least one candidate substance according to the information obtained by the detection or quantification of the expression of the gene above. The information is, for example, quantitative information, and more specifically, the expression level itself of the gene per a cell population having a specific number of cells, the number of photoaged cells obtained by conversion from the expression level, the number of cells in which the expression of the genes is detected, the number of cells in which the expression level is not less than a specific value, and the number of cells in which the expression of the genes is positive.

Further specific aspects of the selection of a specific substance from the candidate substance based on the information include, but are not limited to, the following.
(1) A reference value is determined in advance, and if the quantitative value of photoaged cells of the cells that have been brought into contact with the candidate substance is not more than the reference value, then the candidate substance is selected as a substance that suppresses proliferation or development of a photoaged cell or reduces a photoaged cell.
(2) If the expression level of the gene in the cells that have been brought into contact with the candidate substance is smaller than that in the control cells, then the candidate substance is selected as a substance that suppresses proliferation or development of a photoaged cell or reduces a photoaged cell.
(3) If the proportion of the cells in which the expression of the gene is positive to the total number of cells that have been brought into contact with the candidate substance is smaller than that in the control cells, then the candidate substance is selected as a substance that suppresses proliferation or development of a photoaged cell or reduces a photoaged cell.

Specific examples of the control cells of (2) and (3) include untreated cells which have not been brought into contact with the candidate substance, and cells which have been brought into contact with the candidate substance at a lower concentration.

### [Kit for detecting, quantifying, or sorting out photoaged cell]

One embodiment of the present disclosure includes a kit for detecting, quantifying, or sorting out a photoaged cell, comprising at least one selected from the group consisting of (a) to (c) below:
(a) a primer set capable of amplifying cDNA of at least one gene selected from the group consisting of GPR17, CD34, GABRR1, OR2AG2, CMKLR1, CDH19, CD93, AVPR2, CCR7, and OXGR1;
(b) a probe that specifically hybridizes with mRNA or cDNA of at least one gene selected from the group consisting of GPR17, CD34, GABRR1, OR2AG2, CMKLR1, CDH19, CD93, AVPR2, CCR7, and OXGR1; and
(c) a substance capable of specifically binding to a protein encoded by at least one gene selected from the group consisting of GPR17, CD34, GABRR1, OR2AG2, CMKLR1, CDH19, CD93, AVPR2, CCR7, and OXGR1.

The primer set of (a) above is not particularly limited in sequence, base length, length, chemical modification and the other factors of each primer as long as at least a part of the cDNA from the at least one gene selected from the 10 genes above can be amplified. Each primer can, for example, have a mismatch(es) and/or a gap(s) in the state of hybridizing with the part of the cDNA.

The primer set of (a) is preferably capable of amplifying the cDNA so that the exon-exon boundary of the gene is included.

The primer set of (a) can be used, for example, for qPCR. In qPCR, the length of the amplified fragment is preferably 500 bp or less, 400 bp or less, 300 bp or less, 200 bp or less, 150 bp or less, or 100 bp or less, and can be 50 bp or more.

The length of each primer of the primer set of (a) is, for example, 15 to 40 nucleotides or 17 to 25 nucleotides.

The probe in (b) is not limited in sequence, length, chemical modification and the other factors as long as the probe specifically hybridizes with mRNA or cDNA of at least one gene selected from the 10 genes. For example, the probe of (b) can be a nucleic acid in which at least a part of the phosphate groups is substituted with DNA or RNA; phosphorothioate, methylphosphonate, phosphorodithionate or the like; or a nucleic acid analog such as a peptide nucleic acid (PNA) and a morpholino nucleic acid.

The probe of (b) preferably hybridizes to a part of mRNA or cDNA containing an exon-exon boundary of the gene.

In the present specification, the probe of (b) is preferably linked with a label such as a microparticle, a fluorescent substance, an enzyme, biotin, a radiolabeled substance, and a magnetic particle.

The probe of (b) can be immobilized on, for example, a bead, a substrate, a membrane or the like.

The length of the probe of (b) is, for example, 15 to 40 nucleotides or 17 to 25 nucleotides.

In one embodiment, the probe is, for example, a probe of a microarray capable of detecting or quantifying mRNA or cDNA or an amplified product thereof.

Each primer and the probe can be designed, for example, in consideration of the Tm value and complementarity obtained with a sequence analysis server or software such as Primer-BLAST based on the sequence information of each gene. For the primer or the probe for each of the genes, an existing sequence registered in a database such as various qPCR primer databases can also be used.

The substance of (c) is not particularly limited as long as the substance of (c) is capable of binding to at least a part of the protein encoded by at least one gene selected from the 10 genes above. Such a substance includes, for example, an antibody (polyclonal antibody or monoclonal antibody) or an antibody fragment, and an aptamer whose antigens are these proteins. Examples of the antibody fragment include a Fab fragment, a Fab' fragment, a F (ab')₂ fragment, a Fv fragment, and a ScFv fragment.

The substance of (c) is preferably linked with a label such as a microparticle, a fluorescent substance, an enzyme, biotin, a radiolabeled substance, and a magnetic particle.

In one embodiment, the substance of (c) is an anti-GPR17 antibody, and in a more specific embodiment, the substance of (c) is an antibody whose antigen is Long form of GPR17.

In one embodiment, the substance of (c) is a fluorescently labeled anti-GPR17 antibody, and in a more specific embodiment, the substance of (c) is a fluorescently labeled antibody whose antigen is Long form of GPR17.

For example, the antibody can be prepared by immunizing an animal using a protein encoded by the 10 genes as an antigen, or can be prepared by screening an antibody library such as a phage library. A commercially available antibody can also be used. An antibody fragment can be prepared based on the antibody or genetic information thereof. An aptamer can be obtained by selection from a large-scale library of nucleic acids, peptides and the like by, for example, systematic evolution of ligands by exponential enrichment (SELEX), phage display method, ribosome display, yeast two-hybrid or the like.

More specific and preferable embodiments of the present kit can be understood from the descriptions in [Method for detecting or quantifying photoaged cell] and [Method for sorting out photoaged cell] above.

### EXAMPLES

Hereinafter, the present invention will be described in detail with reference to Examples, but the present invention is not limited to the following Examples as long as the present invention does not exceed the gist thereof.

### [Test Example 1. Preparation of photoaged keratinocyte]

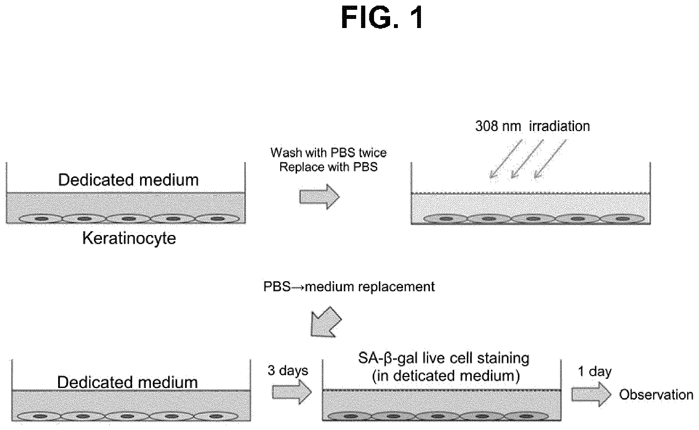

Normal human epidermal keratinocytes (NHEK), which is one type of human skin cells, were irradiated with the ultraviolet ray having a peak wavelength of 308 nm in the following procedure to prepare photoaged cells. Fig. 1 is a schematic diagram of the procedure. Culture and incubation were performed at 37°C using a CO₂ incubator. Cerabeam^{™} UV 308 mini (manufactured by Ushio Inc.) was used as an ultraviolet light source. That is, the irradiated ultraviolet ray is light from a XeCl excimer discharge lamp as a light source that has passed through borosilicate glass (thickness: 1 mm) that cuts a wavelength around 280 nm, and its spectrum is shown in Figs. 8b and 9b of JP-A-2007-267936. The irradiated ultraviolet ray is UVB having no component in a region of less than 297 nm.
(1) NHEKs were cultured in a collagen coated dish (manufactured by Corning Inc.) with a medium dedicated to keratinocytes (manufactured by LONZA) until sub-confluent according to a conventional method.
(2) The medium was removed, the cells were washed twice with phosphate buffered saline (PBS), and then PBS was added so that the cells were immersed adequately.
(3) The ultraviolet ray at 0 mJ/cm², 20 mJ/cm², 40 mJ/cm², 80 mJ/cm² or 100 mJ/cm² was irradiated. Each irradiation dose was obtained by setting the irradiation time to 0, 1, 3, 5, or 6 seconds.
(4) The PBS of the dish was then replaced with a medium dedicated to keratinocytes (manufactured by LONZA KK.) and culture was performed for 3 days.
(5) The medium was removed by suction, 2 mL of SA-β-Gal pretreatment solution (manufactured by Cell Biolabs, Inc.) was added to the medium, and incubation was performed for 2 hours.
(6) The SA-β-Gal pretreatment liquid was removed by suction, 10 µL of SA-β-Gal substrate solution (manufactured by Cell Biolabs, Inc.) was added to the medium, and culture was further performed for 1 day.

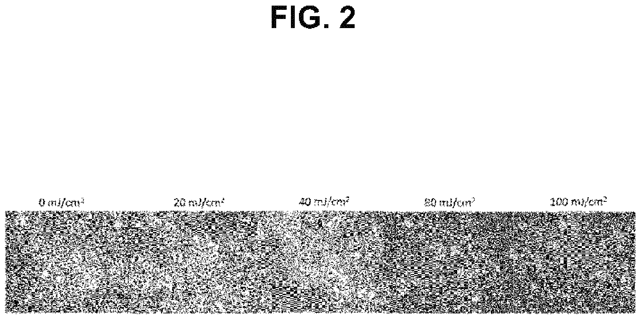

Fig. 2 compares microscopic images of photoaged cells prepared at each irradiation dose. As the irradiation dose increased, the number of cells decreased but detached cells (dead cells) were less observed. This indicates that increased irradiation dose increased the number of cells showing "arrested cell division", which is a characteristic of aged cells. That is, it was found that when the irradiation dose was 40 mJ/cm² or more, the number of cells that had arrested cell division remarkably increased, and when the irradiation dose was 80 mJ/cm² or more, aged cells that had sufficiently arrested cell division were obtained.

When the irradiation dose was increased, the size of cells increased. This represents "cell hypertrophy", which is a characteristic of aged cells.

All of the cells showing the arrest of cell division or the cell hypertrophy were stained with SA-β-Gal, and thus the cells were confirmed to be aged cells.

Thus, it was shown that living photoaged cells can be efficiently prepared by irradiation of the ultraviolet ray having a peak wavelength of 308 nm.

In the case of irradiation with the ultraviolet ray having a peak wavelength of 380 nm, the number of cells killed was small even when the irradiation dose was 100 mJ/cm², whereas in the case of irradiation with the ultraviolet ray having a peak wavelength around 280 nm, about 70% of the cells were killed even when the irradiation dose was 10 mJ/cm². Therefore, it was shown that even when cells were irradiated with the ultraviolet ray having a peak wavelength of 308 nm, the cells were less likely to be killed as compared with normal UVB.

From the above, it is concluded that by adjusting the irradiation dose of the ultraviolet ray having a peak wavelength of 308 nm, the damage given to the cells can be controlled and photoaged cells can be prepared efficiently.

### [Test Example 2. Analysis of mRNA expression in photoaged keratinocytes]

Keratinocytes treated with ultraviolet ray at the irradiation dose of 0 mJ/cm² (without light irradiation) (UN group), at the irradiation dose of 80 mJ/cm² (U80 group) were prepared in the same procedure as in (1) to (5) of Test Example 1. Naturally aged human normal keratinocytes (P50 group) were also prepared by subculturing keratinocytes in a medium dedicated to keratinocytes for 50 days. Each group had n = 10. Cells of each group were collected from the dish, mRNA was extracted from the cells using TRIzol^{™} (manufactured by Thermo Fischer Scientific Inc.), and RNA-Seq analysis was performed. The read counts of all analyzed genes were normalized and compared between groups. The performance of RNA-Seq and the analysis of data were entrusted to DNA Chip Research Inc.

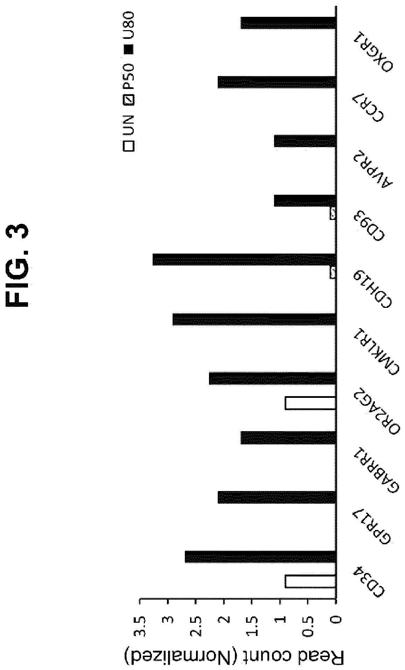

The comparison result of the read counts is shown in Fig. 3. It was found that GPR17, CD34, GABRR1, OR2AG2, CMKLR1, CDH19, CD93, AVPR2, CCR7, and OXGR1 were specifically increased in the photoaged cells prepared by ultraviolet irradiation.

From the above, it was shown that the 10 genes and the proteins thereof can be used as markers for detecting or quantifying photoaged cells.

Among them, mRNA expression of GPR17, GABRR1, CMKLR1, AVPR2, CCR7, and OXGR1 was not observed except for the U80 group. These genes were shown to be markedly specific markers in photoaged cells.

### [Test Example 3. Detection of photoaged cell using GPR17]

Cells irradiated with ultraviolet rays were prepared in the same manner as in Test Example 1 except that a collagen-coated glass plate was used as a cell culture substrate. The irradiation dose was 0 mJ/cm² (UV (-) group) or 80 mJ/cm² (UV (+) group). A collagen-coated glass plate to use was obtained by adding 20 µL/well of 0.1 mL of Cell Matrix Type I-C (manufactured by Nitta Gelatin Inc.) added to 0.9 mL of a diluent (dilute hydrochloric acid pH 3.0) to Multitest slide, 8-well (manufactured by MP BIOMEDICALS), allowing the mixture to stand at room temperature for 30 minutes, and then washing the mixture twice with 20 µL/well of a keratinocyte dedicated medium.

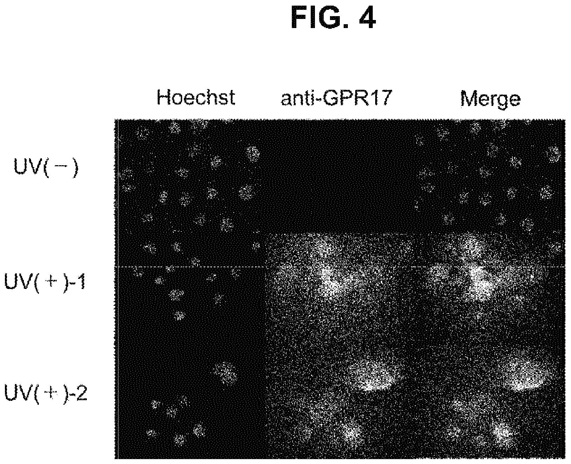

Then, the obtained keratinocytes were stained using a commercially available anti-GPR17 antibody (product code: CSB-PA619758LA01HU, manufactured by CUSABIO BIOTECH Co, Ltd.) having an N-terminal region (residue number 1 to 36) of human GPR17 as an epitope as a primary antibody and Goat anti-Rabbit IgG (H + L) Highly Cross-Adsorbed Secondary Antibody, Alexa Fluor Plus 488 (manufactured by Thermo Fischer Scientific Inc.) as a secondary antibody. Nuclei were also Hoechst stained. The obtained stained images are shown in Fig. 4. Cells in the UV (+) group were stained with anti-GPR17 antibody, while cells in the UV (-) group were not stained. It was shown that because GPR17 is a protein present on the cell surface, photoaged cells are easily stained and can be detected by anti-GPR17 antibody.

Further, by using flow cytometry, the photoaged cells stained with the anti-GPR17 antibody could be easily sorted out.

### [Test Example 4. Preparation of photoaged cell from fibroblast]

Normal human skin fibroblasts (NHDF cells) were irradiated with the ultraviolet ray having a wavelength of 308 nm to prepare photoaged cells. Normal human dermal fibroblasts (NHDF cells) are cells in the dermal region that are one step deeper than human epidermal keratinocyte cells. Experimental conditions other than the cell type and the medium are the same as (1) to (5) in Test Example 1. As the medium, a medium dedicated to fibroblast (manufactured by PromoCell) was used.

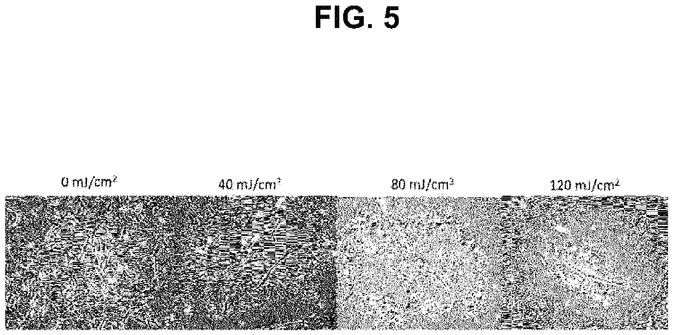

The results are shown in Fig. 5. As with experiments using human epidermal keratinocyte cells, the presence of living cells was confirmed at least at irradiation doses of up to 120 mJ/cm². As the irradiation dose increased, "arrest of cell division" and "cell hypertrophy", which are characteristics of aged cells, were identified. As described above, even when human skin cells other than human epidermal keratinocyte cells were used, photoaged cells could be efficiently prepared by irradiation with the ultraviolet ray having a peak wavelength of 308 nm.

## Claims

1. A method for detecting or quantifying a photoaged cell, the method comprising:
detecting or quantifying expression of at least one gene selected from the group consisting of GPR17, CD34, GABRR1, OR2AG2, CMKLR1, CDH19, CD93, AVPR2, CCR7, and OXGR1 in a cell.

2. A method for sorting out a photoaged cell, comprising:
sorting out a cell positive for expression of at least one gene selected from the group consisting of GPR17, CD34, GABRR1, OR2AG2, CMKLR1, CDH19, CD93, AVPR2, CCR7, and OXGR1.

3. A method for screening a substance that suppresses proliferation or development of a photoaged cell or a substance that reduces a photoaged cell, the method comprising:
bringing a cell into contact with at least one candidate substance;
detecting or quantifying expression of at least one gene selected from the group consisting of GPR17, CD34, GABRR1, OR2AG2, CMKLR1, CDH19, CD93, AVPR2, CCR7, and OXGR1 in the cell that has been brought into contact with the at least one candidate substance; and
selecting a substance that suppresses proliferation or development of a photoaged cell or a substance that reduces a photoaged cell from the at least one candidate substance according to information obtained by the detection or quantification of expression of the at least one gene.

4. A kit for detecting, quantifying, or sorting out a photoaged cell, comprising at least one selected from (a) to (c) below:
(a) a primer set capable of amplifying cDNA of at least one gene selected from the group consisting of GPR17, CD34, GABRR1, OR2AG2, CMKLR1, CDH19, CD93, AVPR2, CCR7, and OXGR1;
(b) a probe that specifically hybridizes with mRNA or cDNA of at least one gene selected from the group consisting of GPR17, CD34, GABRR1, OR2AG2, CMKLR1, CDH19, CD93, AVPR2, CCR7, and OXGR1; and
(c) a substance capable of specifically binding to a protein encoded by at least one gene selected from the group consisting of GPR17, CD34, GABRR1, OR2AG2, CMKLR1, CDH19, CD93, AVPR2, CCR7, and OXGR1.

5. A method for preparing a photoaged cell, the method comprising:
detecting or quantifying expression of at least one gene selected from the group consisting of GPR17, CD34, GABRR1, OR2AG2, CMKLR1, CDH19, CD93, AVPR2, CCR7, and OXGR1 in a cell that has been irradiated with light having an emission peak within a wavelength range of 300 to 315 nm.
